# EUROPEAN PATENT SPECIFICATION

(11) **EP 3 829 435 B1**
(45) Date of publication and mention of the grant of the patent: **18.06.2025**
(21) Application number: 19750177.8
(22) Date of filing: 12.07.2019
(51) Int. Cl.: A61B 5/11, A61B 5/024, A61B 5/029, A61B 5/00, A61B 5/08, A61B 5/16, A61B 5/364

(54) **PROVIDING TEMPORAL INFORMATION OF A SUBJECT**
BEREITSTELLUNG VON ZEITLICHEN INFORMATIONEN EINES SUBJEKTS
FOURNITURE D'INFORMATIONS TEMPORELLES D'UN SUJET

(30) Priority: 27.07.2018 FI 20185659
(43) Date of publication of application: 09.06.2021
(73) Proprietor: Murata Manufacturing Co., Ltd., Nagaokakyo-shi, Kyoto 617-8555 (JP)
(72) Inventor: KINNUNEN, Jere, 00170 Helsinki (FI); MERIHEINÄ, Ulf, 01150 Söderkulla (FI)
(74) Representative: Boco IP Oy Ab
(86) International application number: PCT/IB2019/055952
(87) International publication number: WO 2020/021377

(56) References cited:
- SAMI NURMI: "Nocturnal Sleep Quality and Quantity Analysis with Ballistocardiography", 23 May 2016 (2016-05-23), pages 1 - 57, XP055626917, Retrieved from the Internet <URL:https://aaltodoc.aalto.fi/bitstream/handle/123456789/20858/master_Nurmi_Sami_2016.pdf?sequence=1&isAllowed=y> [retrieved on 20190927]
- INAN O T ET AL: "Robust ballistocardiogram acquisition for home monitoring", PHYSIOLOGICAL MEASUREMENT, INSTITUTE OF PHYSICS PUBLISHING, BRISTOL, GB, vol. 30, no. 2, 16 January 2009 (2009-01-16), pages 169 - 185, XP020153654, ISSN: 0967-3334, DOI: 10.1088/0967-3334/30/2/005

## Description

### Field

The present invention relates to a system and a computer program product related to monitoring a subject, such as a person, and providing temporal information on the subject. More particularly, the invention relates to providing temporal information on a subject that may be used for diagnosing nocturnal arrhythmia based on a single ballistocardiographic signal.

### Background

Making a diagnosis of arrhythmia is easy if it is chronic or persistent, but many times cardiac arrhythmias are episodic in nature - they come and go without warning. In many cases different kinds of cardiac arrhythmia, in particular in an early phase, may only occur during sleep and specifically during certain sleep phases and relaxation states, or not even every night or every week.

Studies of individuals free of cardiac disease show that sinus bradycardia, sinus pauses, and type 1 second degree atrioventricular (AV) block are common during sleep. These are normal and a reflection of changes in autonomic tone that occur during sleep and require no intervention unless accompanied by symptoms.

REM (Rapid Eye Movement) sleep episodes occur approximately every 90 minutes and are associated with the majority of dream activity. Increased brain excitability disrupts the stable autonomic state of non-REM sleep, triggering short bursts of sympathetic neuronal activity that can exceed that of the waking state, and resulting in irregular periods of dramatic hypertension and tachycardia, often associated with disruption of the regular breathing pattern of non-REM sleep. Incidences of non-fatal myocardial infarction, implanted defibrillator discharges, and sudden cardiac death occur in a non-uniform manner throughout the night.

Some conditions significantly increasing the probability of nocturnal arrhythmia are obtrusive sleep apnea and central sleep apnea, with potential fatal conditions such as congestive heart failure or ventricular fibrillation. It has also been suggested, that sudden infant death syndrome is related to nocturnal ventricular arrhythmia.

A nocturnal measurement can thus provide indications of a large variety of cardiac arrhythmias and by relating the measurement results to a simultaneous measurement of the status of the autonomous nervous system, the sleep phase and to the detection of possibly related sleep phenomenon, like obtrusive or central sleep apnea, one can increase detectability of cardiac arrhythmias and thereby facilitate improving of diagnosis.

Ballistocardiography (BCG) is a measure of ballistic forces on the heart. It can be characterized as a mechanical response of the electrocardiographic signal (ECG). As heart pumps blood, two mechanical effects may be measured: motion of the heart causes a recoil effect on the chest, and motion of the blood causes a recoil effect in whole body. A ballistocardiographic (BCG) signal, which may also be called as a ballistocardioglogic signal, has a characteristic form, which is based on the blood flowing up and down in the body. This signal, for example delay and details of the shape of the BCG signal can reveal cardiac dysfunction. So called J-peaks of a BCG signal may be used to measure heart rate (HR) and heart rate variability (HRV) in a similar way as the R-peaks are used in the Electrocardiogram (ECG).

Ballistocardiographic data indicates the extent of mechanical movements of a body that take place in response to the myocardial activity of the heart. Such ballistocardiographic data may then be used to process data that is indicative of heart motion of the subject. Ballistocardiography based on accelerometer(s) or angular rate sensor(s) provides a non-invasive, unobtrusive and relatively lightweight method for measuring both the relative stroke volume of the heart and the beat-to-beat times.

Heart rate variability (HRV) refers to a variation in the beat-to-beat interval of the heart. Although the measured physiological phenomenon is the same for HRV and beat-to-beat interval, typical parameters describing these are different. While beat-to-beat time is expressed typically in time scale, heart rate (HR) is typically expressed on a frequency scale, for example in beats per minute. Heart rate variability (HRV) may be expressed through indicating the relative rate change among a number of consecutive heart beats. Heart rate variability (HRV) may be calculated from detection of beat-to-beat intervals with a suitable data processing function. Variation in the beat-to-beat interval is a physiological phenomenon; the sinoatrial node of the heart receives several different inputs, and the instantaneous heart rate and its variation are results of these inputs. Recent studies have increasingly linked high heart rate variability (HRV) to good health and a high level of fitness, whilst decreased heart rate variability (HRV) is associated to stress and tiredness.

Analysis of heart rate variability (HRV) in the frequency domain is a widely used tool in the investigation of autonomic cardiovascular control. Usually the variability is differentiated in the spectral profile into the high frequency (HF) band (0.10 to 0.40 Hz), the low frequency (LF) band (0.04 to 0.10 Hz), and the very low frequency (VLF) band (< 0.04 Hz). For example, breathing cycle causes a natural, clearly detectable variation of heart rate, where the R-R interval on ECG, and the J-J interval in BCG, is shortened during inspiration and prolonged during expiration. This variation is called Respiratory Sinus Arrhythmia (RSA), which is detected in the high frequency (HF) band. The low frequency (LF) band (0.04 to 0.10 Hz) represents oscillations related to regulation of blood pressure and vasomotor tone including the so-called 0.1 Hz fluctuation. Heart rate variability in the high frequency (HF) band may be referred to as high frequency heart rate variability (HFHRV). Heart rate variability in the low frequency (LF) band may be referred to as low frequency heart rate variability (LFHRV). Correspondingly, heart rate variability in the very low frequency (VLF) band may be referred to as vert low frequency heart rate variability (VLFHRV).

Stroke volume (SV) refers here to a volume of blood pumped from one ventricle of the heart with each beat. The stroke volume may be calculated from measurements of ventricle volumes by subtracting the volume of blood in the ventricle at the end of a beat (called end-systolic volume) from the volume of blood just prior to the beat (called end-diastolic volume). Methods to detect stroke volumes include echocardiograms, ballistocardiographic devices, and pulse wave signal measurements with photoplethysmographs (PPG) or pressure sensors.

Stroke volume variability (SVV) refers to changes in arterial blood pressure induced by mechanical ventilation, which is a physiological phenomenon of variation in the stroke volume in a subject. Stroke volume variability (SVV) is a naturally occurring phenomenon in which the arterial pulse pressure falls during inspiration and rises during expiration due to changes in intra-thoracic pressure secondary to negative pressure ventilation (spontaneously breathing). Stroke volume variability (SVV) may be defined as the percentage change between the maximal and minimal stroke volumes (SV) divided by the average of the minimum and maximum stroke volumes over a floating period.

Respiration rate (RR) refers to rate of respiration of a subject. Although it's not a cardiologic measure, respiration rate (RR) may be detected using the same sensor(s) that are used for detecting ballistocardiographic signals, since respiration causes movement of the body of the subject detectable with accelerometer(s) and/or angular rate sensor(s). For example, respiration rate (RR) may be obtained indirectly from a heart rate signal or a heart rate variation signal by detecting HFHRV variation caused by the breathing cycle. Respiration rate (RR) may also be obtained indirectly from a stroke volume variation (SVV) signal, since stroke volume (SV) signal amplitude is modulated by respiration.

### Description of the related art

Patent FI 126631 B discloses a method to use Heart Rate Variability obtained using ballistocardiography (BCG), that provides an excellent tool for estimating stress, nocturnal recovery and sleep quality.

Patent FI 126600 B discloses that BCG may also be used to detect sleep apnea.

A M.Sc. thesis document "Nocturnal sleep quality and quantity analysis with ballistography" by Sami Nurmi for Aalto University evaluates usability and performance of a ballistocardiography based method for qualitative and quantitative analysis of sleep. The document specifically describes a system wherein a plurality of BCG sensor nodes are positioned under a bed mattress. An algorithm associated with the BCG sensor nodes is then able to report multiple parameters, namely timestamp, heart rate, respiratory rate, relative cardiac stroke volume, heart rate variability, signal strength, bed occupancy status and beat-to-beat intervals.

"Robust ballistocardiogram acquisition for home monitoring" by O.T. Inan, M. Etemadi, R.M. Wiard, L. Giovangrandi and G.T.A. Kovacs of Stanford University discloses a method for acquiring BCG signals.

BCG measurement is non-intrusive, and it has little or no effect on sleep and relaxation, and it is therefore suitable for continuous long-term monitoring. Thus, BCG overcomes many problems related to detection of sleep phases and sleep apnea.

A state of the art way to measure nocturnal arrhythmias is with a 24 hour Holter measurement using a recorder and electrocardiography (ECG) electrodes attached to the chest of the patient. The ECG measurement is not very convenient, but intrusive for the patient and thus may distort the sleep phases with a potential consequence of not showing arrhythmia that would exist during a normal night. Artefacts caused by the person moving may cause false arrhythmia detections and are difficult to filter out from a recorded ECG signal. Further, Holter measurement entirely lacks data on sleep phases.

A standard way today to relate nocturnal arrhythmia to a specific sleep phase is by using polysomnography (PSG). PSG is a multi-parameter sleep study that comprises monitoring many body functions including brain, eye movements, muscle activity or skeletal muscle activation and heart rhythm during sleep. Typical polysomnography uses a broad range of invasive detection methods including electroencephalography (EEG), electrooculography (EOG), electromyography (EMG) and ECG. However, this is extremely intrusive and thus influences the stress level and the sleep of the patient. A night during polysomnography is very different from a normal night at home, and the patient may not at all go into sleep phases, where arrhythmia would exist.

Some measurements for polysomnography may also be performed using a pressure sensor under or within a mattress under the person that can track heart rate, breathing and movement by sensing changes in pressure under the patient.

Patent application US 2018049701 A1 discloses a mattress for resting or sleeping of a person that includes a sensor arranged in connection with the mattress for determining the heart rate, the heart rate variation, breathing disturbances, the respiratory frequency and/or the categories of sleep.

However, pressure-based sensor devices are not capable of detecting for example stroke volume and/or stroke volume variation, which may be important factors in determining whether arrhythmias occur. A device and a method are therefore needed to detect nocturnal arrhythmias non-invasively.

### Summary

An object is to provide a computer program product and apparatus so as to solve the problem of detecting nocturnal arrhythmia in non-invasive manner. The objects of the present invention are achieved with a system according to claim 1. The objects of the present invention are further achieved with a computer program product according to claim 6.

The preferred embodiments of the invention are disclosed in the dependent claims.

The present invention is based on the idea of detecting different types of nocturnal arrhythmia based on beat-to-beat times and relative stroke volume of the heart that may be obtained from a single ballistocardiographic signal obtained non-invasively from a subject. The same ballistocardiographic signal may further be processed to provide information on sleep disorder, stress, recovery and/or sleep phases, which enables detecting possible relationships between the detected arrhythmia and the above mentioned physiological phenomena occurring during sleep. Signals obtained from the single ballistocardiographic signal are processed to filter out any artefacts in the signal which could introduce erroneous detection results.

According to a first system aspect, a nocturnal arrhythmia detection system is provided according to any one of claims 1 to 5.

According to another aspect, a computer program product configured to perform steps of the method of any one of claims 6 to 10.

The present invention has the advantage that the method enables reliable detection and identification of various types of arrhythmia with a simple, durable, easy to install, lightweight and cost-efficient system, while the measurements do not affect sleep of the subject. By combining obtained arrhythmia information with information on the persons movements, sleep stages, recovery stages, sleep disorder and/or stress received simultaneously with the same detection system, the method and the system may facilitate a more detailed and accurate diagnosis by a medical practitioner receiving the information.

### Brief description of the drawings

In the following the invention will be described in greater detail, in connection with preferred embodiments, with reference to the attached drawings, in which
Figure 1 illustrates an exemplary configuration of a monitoring system.
Figure 2 shows an exemplary method of indicating occurrence of cardiac arrhythmia
Figure 3 illustrates a method of indicating movement of the subject.
Figure 4 illustrates an embodiment of filtering the TB2B signal.
Figure 5 illustrates an embodiment of filtering the SV signal.
Figure 6 illustrates a plot showing detection signals obtained by processing a single BCG signal.
Figure 7 another a plot showing detection signals obtained by processing a single BCG signal.
Figure 8 illustrates signal strength and beat-to-beat times during a detected arrhythmia.
Figure 9 illustrates variation of beat-to-beat times during detected arrhythmia.

### Detailed description

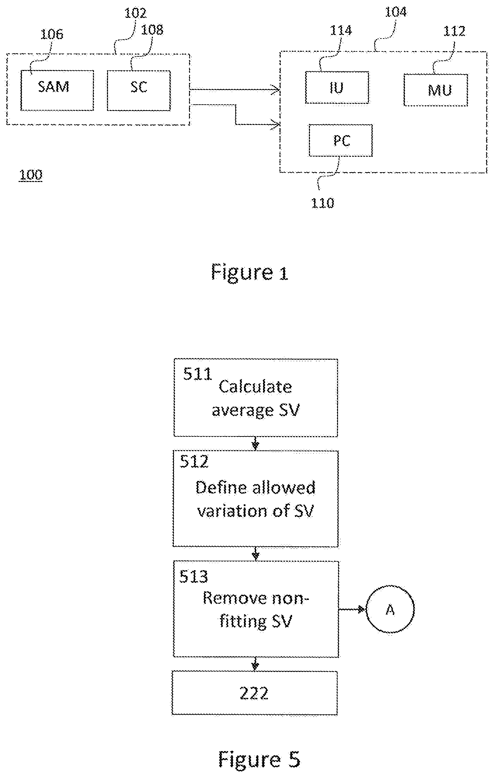

Figure 1 illustrates an exemplary configuration where the monitoring system (100) comprises a sensor unit (102) and a control unit (104). The sensor unit (102) may be considered as an element to be attached to the monitored subject and the control unit (104) may be considered as an element communicatively coupled to the sensor unit (102) but physically detached from the monitored subject. The sensor unit (102) may be directly attached to or pressing on the monitored subject, or it may be placed to indirectly obtain a ballistocardiographic signal from an element attached to or pressing on the subject, e.g. a bed or a seat. The control unit (104) advantageously comprises circuitry adapted to process signal provided by the sensor unit (102) and circuitry to process data obtained from the signal.

The sensor unit (102) includes one or more sensors (106) for obtaining a ballistocardiographic signal. Ballistocardiology refers in general to a technology for measuring movements of a body, which are caused in response to shifts in the center of the mass of the body during heart beat cycles. The sensor may sense linear or angular motion of the body and thus be, for example, an accelerometer, or a gyroscope. In the context of the claimed invention, the sensor unit comprises at least an accelerometer as sensor.

The sensor unit (102) may also include a signal processing unit (108) that manipulates the raw electrical input signal to meet requirements of a next stage for further processing. Signal processing may include, for example, isolating, filtering, amplifying, and converting a sensor input signal to a proportional output signal that may be forwarded to another control device or control system. A signal processing unit (108) may also perform some computation functions such as totalization, integration, pulse-width modulation, linearization, and other mathematical operations on a signal. The signal processing unit (108) may alternatively be included in the control unit (104).

The control unit (104) is a device that comprises a processing component (110). The processing component (110) is a combination of one or more computing devices for performing systematic execution of operations upon predefined data. The processing component may comprise one or more arithmetic logic units, special registers and control circuits. The processing component may comprise or may be connected to a memory unit (112) that provides a data medium where computer-readable data or programs, or user data can be stored. The memory unit may comprise one or more units of volatile or non-volatile memory, for example EEPROM, ROM, PROM, RAM, DRAM, SRAM, firmware, programmable logic, etc.

The control unit (104) may also comprise or may be connected to an interface unit (114) that comprises at least one input unit for inputting data to the internal processes of the control unit, and at least one output unit for outputting data from the internal processes of the control unit. The output unit may comprise or may be coupled with at least one display unit configured to present data provided by the control unit.

If a line interface is applied, the interface unit (114) typically comprises plug-in units acting as a gateway for information delivered to its external connection points and for information fed to the lines connected to its external connection points. If a radio interface is applied, the interface unit (114) typically comprises a radio transceiver unit, which includes a transmitter and a receiver. A transmitter of the radio transceiver unit may receive a bit stream from the processing component (110) and convert it to a radio signal for transmission by an antenna. Correspondingly, the radio signals received by the antenna may be led to a receiver of the radio transceiver unit, which converts the radio signal into a bit stream that is forwarded for further processing to the processing component (110). Different line or radio interfaces may be implemented in one interface unit.

The interface unit (114) may also comprise a user interface with a keypad, a touch screen, a microphone, or equals for inputting data and a screen, a display, a touch screen, a loudspeaker, or equals for outputting data to a user of the device, when triggered by detection of a cardiac arrhythmia. Data to a user may be used for providing a monitoring result such as displaying or sounding an indicator by the interface unit (114) or causing an alarm to be initiated on the interface unit (114). In addition to instantaneous presentation of detected variables, the interface unit (114) or a memory device attached to it (not shown) may store the data for later display and even further analysis.

The processing component (110) and the interface unit (114) are electrically interconnected to provide means for performing systematic execution of operations on the received and/or stored data according to predefined, essentially programmed processes. These operations comprise the procedures described herein for the control unit of the monitoring system of Figure 1.

The sensor unit (106) for obtaining a ballistocardiographic signal may comprise an accelerometer or an angular rate sensor (gyroscope). In the context of the claimed invention, the sensor unit comprises at least an accelerometer.

In case an angular rate sensor, such as a gyroscope, is used, the sensor unit is advantageously attached to the chest of the subject from which the rotational movement of the heart at every heart beat can be detected and obtained.

In linear detection, the sensor unit (106) may be attached directly to the subject, but the sensor unit is preferably attached to the subject indirectly, for example to a bed where the subject rests in, a mattress in the bed, or the like. The sensor unit (106) may be an accelerometer that detects the recoil signal of the blood moving in the arteries from the movement transferred to the intermediate item (e.g. the bed or mattress) from the body. Attaching or placing the sensor unit in the bed is beneficial, since it enables a very natural sleeping environment, since no sensors or other measurement devices need to be attached to the subject. Thus, the measurement situation itself likely causes less stress on the subject, and the results achieved from the measurement correspond to a natural sleeping situation of the subject.

When an accelerometer is used as the sensor unit (106) detecting a ballistocardiographic signal from a person (as is the case in the claimed invention), the most important aspect is measuring acceleration in the longitudinal dimension of the subject. Measurement of acceleration along a single axis may be implemented with a single one-axis accelerometer as long as the accelerometer is disposed in a position and orientation that the detection axis of the accelerometer is substantially aligned with the length (the longitudinal axis) of the subject. For example, an accelerometer may be installed to a bed structure so that it measures acceleration in the longitudinal dimension of the bed. When the subject lies in the bed longitudinally, the accelerometer may measure acceleration caused by the recoil signal of the moving blood. The received ballistocardiographic acceleration signal may be assumed to be strongest in the longitudinal direction of a human subject.

Instead of the above mentioned ballistocardiographic devices, in a different configuration useful to understand the claimed invention, a signal that is indicative of both stroke volumes and beat-to-beat times of the heart of a subject can alternatively be obtained with a pulse wave measurement device. Such device comprises a fastening element for detachably attaching a pressure sensor to a position on the outer surface of a subject. The sensor unit (106) may thus be a pressure sensor configured to generate a pulse wave signal that varies according to deformations of the tissue in response to an arterial pressure wave expanding or contracting a blood vessel underlying the tissue in the position. The signal processing unit (108) may be configured to receive the pulse wave signal and compute from it pulse wave parameters that represent stroke volumes and beat-to-beat times of the heart of a subject.

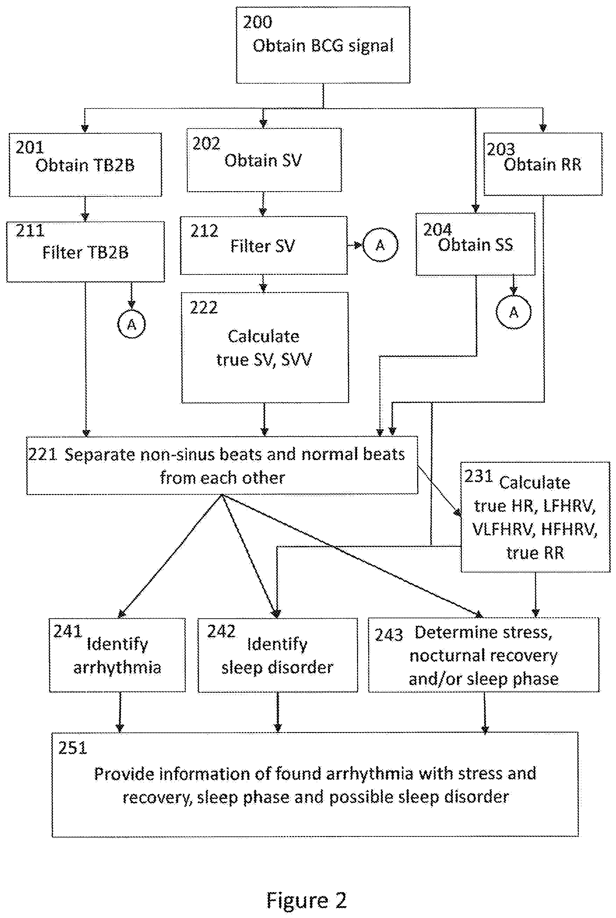

The figure 2 shows an exemplary method of indicating occurrence of cardiac arrhythmia.

The method starts at phase 200 by obtaining a BCG signal. The BCG signal may be obtained from a subject with a single BCG device. The obtained BCG signal is then processed for obtaining a number of variables. A beat-to-beat time (TB2B) signal is obtained by detecting the J-peaks within the signal in the phase 201. In addition, a heart rate (HR) signal, which represents inverse of the beat-to-beat time, may be obtained on basis of the J-peaks in the BCG signal. The TB2B signal is used for further processing and detecting for example arrhythmia, but also the heart rate signal (HR) may be provided as a parameter that may be used for facilitating diagnosis.

A stroke volume (SV) signal is obtained by processing the same obtained BCG signal in the phase 202 that was processed for obtaining TB2B and optionally HR signal.

For reliable and robust results, the SV signal is preferably not an instantaneous measurement result but represents a sum of absolute values of multiple BCG measurements calculated during different phases of a single heart beat period. In one particular example, the SV signal represents a sum of absolute values of four consecutive measurements made during a single heart beat at specific phases of the heart beat process. By calculating the sum of different phases of the acceleration of the body of the subject caused by ballistocardiologic forces resulting from changes of blood stream during the various heart beat phases rather than just a single peak, the obtained SV signal is more robust and less susceptible to noise and offset errors. Alternatively, a single peak of the ballistocardiographic signal could be used as a representative of stroke volume, although such stroke volume measurement is prone to noise and offset errors.

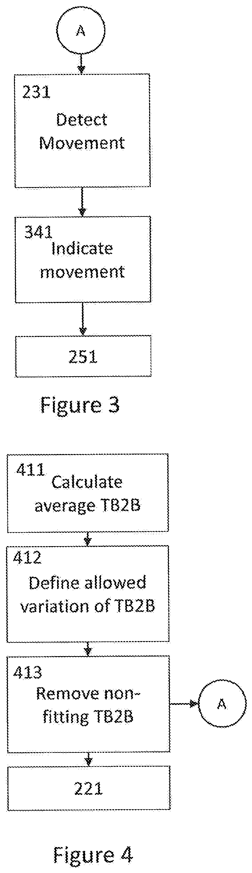

Further, a respiratory rate (RR) signal may be obtained from the same obtained BCG signal in the phase 203. The RR signal is a secondary signal, which can be obtained for example by detecting changes in the TB2B over the breathing cycle. The RR signal is not needed for arrhythmia detection, but it may be used for other purposes, for example sleep disorder detection and determining stress, nocturnal recovery and/or sleep phase. Another useful value that is obtained from the BCG signal is signal strength. This is illustrated with the phase 204. Signal strength (SS) in the context of the claimed invention is a signal that represents the overall average root mean square strength of the received BCG signal during a time period. The SS signal may be calculated for example over a sliding window. Signal strength may be calculated over a sliding window of 0.1 to 10 seconds. In one exemplary embodiment, the SS signal presents root mean square of the overall signal strength during a 1 second period. The SS signal may be used, for example, in making decisions whether the obtained BCG signal indicates that the subject is moving. For this purpose, the SS signal is fed towards a movement detection process illustrated in the figure 3 as illustrated with the output "A" from the phase 204.

The SS signal is used as a discriminating signal when making decisions on whether received signals represent just background noise, actual detected BCG signals or movement of the subject. A very weak signal strength, indicated by a low SS signal value, may indicate, that the received measurement results may just be caused by noise, such as vibration caused by the environment, and that the subject is not actually present to produce any real BCG signals. On the other hand, a very strong signal strength, in other words a high SS signal value, may indicate that the subject is moving, which causes much stronger signals to be received than what would be caused by actual BCG signals. When the signal strength remains within a predefined signal strength interval, it is likely that the obtained signal actually represents actual BCG measurement results. The SS signal may thus be used for motion detection as well as for separating motion artefacts, non-sinus beats and normal beats from each other. The predefined signal strength interval may be adjusted on basis of size of the subject as well as the posture of the subject.

Preferably, phases 201, 202, 203 and 204 are performed substantially simultaneously. In addition to obtaining TB2B, SV, SS and the optional RR and HR signals as disclosed in the phases 201, 202 203 and 204, further signals may be obtained from the BCG signal, such as a high frequency heart rate variability (HFHRV) signal and a status signal (ST). Large HFHRV, in other words large TB2B variation, in combination with strong SS signal also indicates motion. By using the HFHRV in combination with SS signal, wrong "beats" in the BCG signal caused by motion rather than actual heart beats may be detected with good accuracy. On the other hand, high HFHRV combined with SS signal within the expected signal strength interval serves as an indicator of arrhythmia. In an embodiment, a status signal (ST) may be used as a simplified, stepwise indication of strength of the SS signal in relation to calibration parameters. The status signal (ST) may be calibrated to have particular values, when the SS signal is below normal, within the normal range or above normal signal strength. Status signal may thus be used for example to adjust settings used in signal processing. Use of status signal (ST) improves separability of motion and actual heart beats.

In the phase 211, the TB2B signal is further filtered for obtaining a filtered TB2B signal. With this filtering, any erroneous peaks in the TB2B signal that are not real J-peaks caused by heart beats are recognized and filtered out. For example, movements of the subject may cause peaks in the TB2B signal that are stronger than any of the actual J-peaks caused by heart beats. In other words, the filtered TB2B signal is free from any motion artefacts. On the other hand, some external sources such as traffic in a nearby street or a pump device operating nearby, may cause peaks in the TB2B signal, but typically such noise peaks are weaker than the actual J-peaks. In one embodiment, at least some of the TB2B peaks that were filtered during the TB2B filtering phase 211 may be further processed for example in order to recognize times at which the subject is moving. Forwarding information on filtered, erroneous peaks in the TB2B signal towards movement detection process is illustrated with the output "A" from the phase 211.

In the phase 212, the SV signal is further filtered for obtaining a filtered SV signal. With this filtering, any erroneous values in the SV signal that are not true SV values caused by heart beats are recognized and filtered out. In other words, the filtered SV signal is free from any motion artefacts. For example, movements of the subject may cause peaks in the SV signal that are stronger than any of the actual SV signal. However, some of the very strong SV signal values may possibly indicate an exceptionally large stroke volume. On the other hand, some external sources such as traffic in a nearby street or a pump device operating nearby, may show in the SV signal, but typically such noise origin signal values are weaker than the actual SV signal. In one embodiment, at least some of the SV readings that are filtered out during the SV filtering phase 212 may be further processed for example in order to recognize times at which the subject is moving. Forwarding information on filtered, erroneous peaks in the SV signal towards movement detection process is illustrated with the output "A" from the phase 212. Preferably such exceptional SV readings are used for detecting movement together with the out-filtered high TB2B peaks obtained from the phase 211.

In the phase 222, a true stroke volume (SV) signal and a stroke volume variability (SVV) signals are calculated based on the filtered SV signal. The true SV signal is now substantially free from errors caused by erroneous values not caused by heart beats, and thus reflects more reliably the actual instantaneous stroke volume and stroke volume variation of the subject.

In the phase 221, a separation of normal and non-sinus heart beats is performed. A normal beat-to-beat time of the subject may be defined for a particular time period. This time period is adjusted not only based on the subject, but it may also be adjusted depending on normal variation of the beat-to-beat times for example due to respiration. It is well known that sinus arrhythmia causes beat-to-beat variation, and the amount of respiratory sinus arrhythmia varies between people depending for example on their age, health and physical condition. On the other hand, lack of sinus arrhythmia or sinus arrhythmia not correlating to respiration rate are possible indicators of health problems.

Apart from the TB2B signal and optionally the true HR signal, the true SV and the SVV signals may be used as additional parameters in determining whether arrhythmia is normal respiratory sinus arrhythmia or an abnormal condition. For example, continuous arrhythmia may decrease average stroke volume, and it often increases stroke volume variability, especially if the arrhythmia comprises late beats, which cause the heart to be filled with more blood during the beat, and the stroke volume thus increases, whereas the next beat which may follow after a shorter than normal period, which causes the stroke volume to be smaller for the next beat.

By analyzing the true TB2B signal, any individual abnormal heart beats, such as non-sinus beats, may be recognized that do not fall within the allowed variation of beat-to-beat times and separated from the normal heart beats. A normal TB2B is defined for the subject for each time instant. The normal TB2B is preferably defined for a limited length time period, so that the normal TB2B varies over the night. Such time period should preferably be slightly longer than the longest breathing cycle of the subject. If the time period is too much longer than the breathing cycle, heart beats may be erroneously identified as non-sinus beats when respiration rhythm changes. For example, the time period used for defining normal TB2B could be about 10 seconds. A time window, in other words a period of time, is defined based on this normal TB2B, during which next normal beat is expected after the previous one. The time window is preferably personally adapted for each subject, and the time window may also be adapted during the measurement period, depending for example on current sleep phase, respiration rate and so on. In one embodiment, the time window is defined dynamically based on historical HRV data of the subject. Adaptation of the time window is important to ensure that only the actual abnormal beats are identified as such.

If the obtained true TB2B is shorter or longer than the defined time window, the beat is identified as being an abnormal beat. Also continuous and/or persistent arrhythmia may be separated from normal heart beats during the phase 221.

In the phase 231, a true heart rate (HR) signal is calculated based on the filtered TB2B signal after removing any motion artefacts. The true HR signal is now substantially free from errors caused by erroneous peaks not caused by heart beats, and thus reflects more reliably the actual instantaneous heart rate of the subject. Further, a low frequency heart rate variability (LFHRV) signal and a very low frequency heart rate variability (VLFHRV) signal may be calculated in the phase 231 based on the filtered TB2B signal after removing non-sinus beats in the phase 221. Also, a true respiration rate (RR) signal may be obtained during the phase 231 that is free from any motion artifacts. The true RR signal is preferably obtained from a signal or signals from which both motion artefacts and any non-sinus beats have been separated in the phase 221.

As a result of the separation phase 221, two sets of beats are obtained. The abnormal beats may be utilized for identifying type of arrhythmia and times or time periods of occurrence of the arrhythmia in the phase 241. Type of arrhythmia may be identified on basis of abnormal TB2B signal and abnormal SV signal. SS signal is preferably used to ensure that only signals caused by real heart beats are included in the analysis. Abnormal stroke volume reflects amount of time available for the heart to fill before the heart beat. Thus, abnormal stroke volume may be smaller than normal when TB2B is shorter than normal, or stroke volume may be greater when TB2B is longer than normal. The normal beats received from the phase 221 may be utilized for identifying occurrences of sleep apnea in the phase 242 and for calculating stress and nocturnal recovery as well as determining sleep phase in the phase 243. Sleep apnea may be determined in the phase 242 on basis of changes in HFHRV, LFHRV, VLFHRV and at least one of RR and true RR signals. For example, sleep apnea may cause decreased HFHRV, increased LFHRV and VLFHRV and higher variation of RR, when compared to same signals during normal sleep. In some cases, also increased SS signal may indicate sleep apnea, for example in case of restless legs, snoring or epilepsy. Sleep phase, stress and recovery may be determined in the phase 243 on basis of HFHRV, LFHRV or SVV. A smaller or decreasing HFHRV, LFHRV and/or SVV may indicate stress. Increasing HFHRV, LFHRV and/or SVV indicates recovery. Sleep phases may be detected from changes in the HFHRV, LFHRV and/or SVV together with RR or RR variation. Also SVV indicates depth of breath and changes in it.

Many types of continuous or persistent arrhythmia may be identified from the true HR signal by extrapolating, correlation and other statistical methods. A combination of the true HR, SV, SVV and raw HFHRV may be used for the identification process. Raw HFHRV refers to variation of beat-to-beat times TB2B between consecutive beats. For example, these include, but are not limited to:
- tachycardia, higher than average HR when subject is in rest
- bradycardia, lower than average HR which may also include missing beats
- very small sinus arrhythmia or SVV, which may be an indicator of dehydration or diabetes
- atrial fibrillation, in which the upper chambers of the heart beat irregularly (quiver)
- atrial flutter causing the atria to beat excessively fast
- ventricular fibrillation, in which the heart quivers instead of pumping due to disorganized electrical activity in the ventricles

Further, the filtered TB2B signal allows detecting occasional arrhythmia, such as heart blocks causing just single or few left out heart beats, premature ventricular contractions that appear as extra heart beats or other types of pathological intervals between heart beats.

When any type or arrhythmia is identified in the phase 241, temporal information on the occurrence of arrhythmia is provided for the phase 251. Term temporal information on a phenomenon refers to information that ties the occurrence of a phenomenon to time, such as an instant or a period (interval) of time. The temporal information on arrhythmia preferably comprises identification of the type of arrhythmia received from the identification phase 241 and timing information thereof. For continuous or persistent arrhythmia, the temporal information may comprise the type of arrhythmia together with information on a period or periods of time during which this type of arrhythmia appeared, and for occasional arrhythmia, for example a bradycardia (missing beats) or extra beats, the temporal information may comprise the type of arrhythmia together with information on one or more time instances of the occurrence of this particular arrhythmia.

In parallel with identifying arrhythmia in the phase 241, sleep disorders such as different types of sleep apnea may be detected and identified in the phase 242, and a temporal information of the detected sleep disorder is provided as an output. The temporal information of sleep disorder thus comprises type(s) of detected sleep disorder and the time instance(s) or period(s) of occurrence of those. An example of using BCG signal as basis for detecting sleep disorders is disclosed in the patent FI 126600 B, according to which sleep disorders, such as sleep apnea may be identified based on at least one of LFHRV and VLFHRV, RR and SVV signals. The temporal information on detected and identified sleep disorders may then be combined with the temporal information on arrhythmia in the phase 251, so that a medical practitioner may perform analysis based on the combined temporal information. Combining the temporal information on sleep disorders with temporal information on arrhythmia facilitates an improved diagnosis on the arrhythmia and related physical phenomenon.

Further, in parallel with the phases 241 and 242, signals obtained in phases 221 and 231 may be used to determine temporal information on stress, nocturnal recovery and/or sleep phase of the subject in the phase 243. For example, patent FI 126631 B discloses a method to use heart rate variability obtained using BCG measurements for estimating at least one of stress, nocturnal recovery and sleep quality based on parameters that comprise at least the RR signal, the HFHRV signal and the LFHRV signal. Preferably, true RR, HFHRV and LFHRV signals are used for this determining that are free from motion artefacts and also free from non-sinus beats. Alternatively, RR signal obtained in the phase 203 and HFHRV and LFHRV signals obtained on basis of filtered TB2B may be used. Temporal information of stress, recovery and/or sleep phase at each point of time may be preferably provided in combination with temporal indicators of arrhythmia in the phase 251. Providing the temporal information on stress, nocturnal recovery and/or sleep quality in combination with temporal information on arrhythmia further facilitates an improved diagnosis on the arrhythmia and related physical phenomenon. Providing information in the phase 251 may comprise for example presenting the temporal information on a display device and/or storing the temporal information on a memory device and/or transmitting the temporal information.

According to one embodiment, the original, unfiltered HR signal obtained in the phase 201 may be compared to the true HR signal or even to a normalized HR signal, namely the true HR signal comprising only normal heart beats, in other words from which any changes in the HR due to arrhythmia have been removed in the phase 231. This kind of comparisons between different indicators of heart rate may provide further information for a medical practitioner using the data received from the device for making an improved diagnosis.

Figure 3 illustrates use of the TB2B peaks and/or SV values filtered out in the phases 211 and 212 for detecting and indicating movement of the subject for providing as temporal information on movement of the subject. High peaks in the TB2B signal are likely caused by movement of the subject so that the time and strength of the out filtered high TB2B peaks may be used as an indicator of movement of the subject. Thus, movement detection in the phase 341 may be based on detection of instances of high TB2B peaks that are filtered out, and the time instances and/or periods of such out-filtered TB2B filters may be referred to as temporal information on movement of the subject. When the subject is moving, SS signal is high, in other words the root mean square average of the acceleration signal increases, likely above its predefined signal strength interval. If the movement and thus the amplitude received from the accelerometer remains within an allowed range, stroke volume and HFHRV that indicates TB2B variation will increase. Amplitude of the BCG signal may be compared to a set of calibration parameters for evaluating whether the measured acceleration amplitude remains in an allowed range. In other words, the allowed range for acceleration signal amplitude may be defined with a set of calibration parameters. Further, filtered values of SV signal may be used together with the TB2B peaks for determining movement of the subject. If the subject moves, SS increases. If the movement is within acceptable range in comparison with the measurement parameters, SV signal and HFHRV, in other words variation of the TB2B increase. The range may be defined for example using the stepwise ST signal. The obtained temporal information of movement may be combined in the phase 251 together with temporal information of arrhythmia, sleep disorder, stress, recovery and/or sleep phase.

All obtained signals, processed data, received calculation results and/or data output as indications shown in the figures 2 and 3 or otherwise mentioned above may be stored in the memory unit of the monitoring system as well as provided at the interface unit. For example, a display of the interface unit or a display directly or indirectly coupled to the interface unit may show a timeline of the night with indicators of detected arrhythmia together with indicators of stress, recovery, sleep cycles and sleep apnea. Any type of obtained data, including but not limited to the raw signals, processed signals and resulting temporal information on the various phenomena, may also be stored to a removable memory device for transferring the data, printed out with a printer, or provided at a local or a remote user interface for example as graphical or textual output.

Figure 4 illustrates an embodiment of filtering the TB2B signal. The overall strength of the TB2B signal typically varies depending for example the posture of the subject. Thus, the TB2B signal needs to be analyzed over a limited period of time to make the analysis adaptable to chances in the signal strength. In the phase 411, an average root mean square signal strength for the TB2B over a suitable moving time window is calculated, wherein the time window corresponds to a time period that is preferably longer than the period of a breathing cycle of the subject. Period of the breathing cycle may be obtained from the RR signal. In the phase 412, an allowed variation range for TB2B signal strength is defined for the J-peaks around the average TB2B signal strength. Each peak in the TB2B signal may be compared to this average. Further, for more reliable detection of J-peaks, SV signal may be used together with TB2B signal strength in the TB2B filtering process. In the phase 413, any TB2B peaks with signal strength outside the allowed signal range are considered as erroneous peaks, in other words not true J-peaks, and are filtered out. The filtered TB2B signal is then provided as input to the phase 221. The out-filtered TB2B signal values with signal strength above the allowed signal range may be used for movement detection as indicated by the output "A". Not only very strong peaks are filtered out by this filtering, but also weak peaks having signal strength below the allowed variation range may be filtered out. However, only TB2B signal peaks that are clearly stronger than expected are preferably used for movement detection.

Figure 5 illustrates an embodiment of filtering the SV signal. The overall strength of the SV signal typically varies depending for example the posture of the subject. Thus, the SV signal needs to be analyzed over a limited period of time to make the analysis adaptable to chances in the signal strength. In the phase 511, an average root mean square signal strength for the SV over a suitable moving time window is defined, wherein the time window corresponds to a time period that is preferably longer than the period of a breathing cycle of the subject. Period of the breathing cycle may be obtained from the RR signal. In the phase 512, an allowed SV signal strength variation range for SV signal strength is defined around the average SV signal strength. The obtained SV signal is then compared with this average. Any detected SV signal strength outside the allowed signal range may be considered to indicate an erroneous result, in other words not true SV reading, and may be filtered out in the phase 513. The filtered SV signal is then provided as input to the phase 222. Not only very high values caused by strong peaks in the BCG signal are filtered out by this filtering, but also weak peaks having signal strength below the allowed variation range may be filtered out. The out-filtered SV signal values above with signal strength above the allowed variation range may be used for movement detection as indicated by the output "A". However, only SV signal peaks that are clearly stronger than expected are preferably used for movement detection.

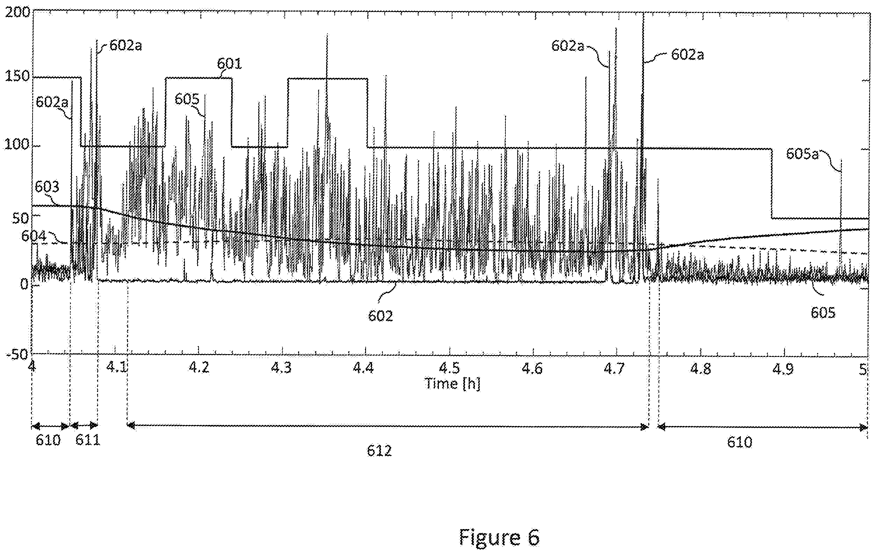

Figure 6 illustrates an example of a plot showing selected signals obtained by processing a single BCG signal according to the invention. In a real-life monitor or a paper plot, different curves representing obtained signal values may be presented for example with different colors, which makes the output more visual. The X-axis is a time axis that represents a period of one hour. The Y-axis represents an arbitrary scale, which may be defined differently for different detected signals. For example, the HRV curve (605) and the signal strength curve (602) preferably have mutually different scales, which facilitates visual distinction between the characteristics of interest of the signals.

Step-like sleep phase curve (601) represents detected sleep phases. Value 150 of the sleep phase curve (601) indicates deep sleep, value 100 indicates shallow sleep, and value 50 represents REM sleep and value 0 indicates that the subject is awake.

Movement of the subject is indicated with the signal strength curve (602), that may be used as an indicator of subject movements. The subject mainly sleeps without moving during this exemplary period, but few clear peaks (602a) can be identified that indicate that the subject has moved, which causes strong peaks. Such peaks (602a) indicate need for filtering obtained SV and TB2B signals in order to remove movement origin artifacts.

Solid line stroke volume (SV) curve (603) indicates filtered stroke volume and dashed line filtered HRV curve (604) indicates a filtered heart rate variation after filtering HRV caused by arrhythmia away. The stroke volume curve (603) in this plot is formed by filtering the SV signal with an exponential filter, that may use for example equation y(t) = (1-k)*y(t-1) + k*(x(t)). True HRV is indicated by a HRV curve (605).

Based on the measurement results, the exemplary period of one hour may be subdivided into different periods, some of which are marked below time axis. During the first and the last periods (610), the HRV curve shows relatively small variation, and amount of movement is also small, so that these periods may be assumed to represent stages of peaceful sleep with normal heart beat. Some natural HRV occurs for example due to respiratory heart rate variation. During these periods of normal heart beat, the stroke volume curve (603) representing true stroke volume signal, from which any artifacts caused for example by movement have been filtered, marked with a smooth solid line, remains in rather steady level, and the filtered HRV curve (604), marked with a dotted line and representing HRV from which any abnormal beats are filtered away, remains fairly stable. However, a single high peak (605a) can be detected in the true HRV curve, which may indicate an occasional abnormal heart beat or two.

Period 611 illustrates changes in signal levels due to movement of the subject, which also causes increase of both the strength and variation of the true HRV signal. However, high overall signal strength (high SS) suggests, that it is likely that this highly varying true HRV signal is not caused by arrhythmia, but movement of the subject causes erroneous detection. In other words, signals that would otherwise appear as an abnormally short TB2B or an abnormal SV signal may simply be caused by movement of the subject. In addition to or in combination with abnormal HRV, movement of the subject can be detected by tracking the overall signal strength (SS signal).

Period 612 illustrates a period with high true HRV, but the movement of the subject is very small, since the signal strength (602) remains constantly substantially zero in the scale used for the signal strength signal (602). The high level and variation of the true HRV signal (605) during this period gives a clear indication of persistent or continuous arrhythmia. Also, the SV signal (603) values are lower than during the periods of normal heart beat (610), which indicates that the heart beats during this period do not produce the normal stroke volume. This information further confirms that the changes in the HRV signal is caused by arrhythmia.

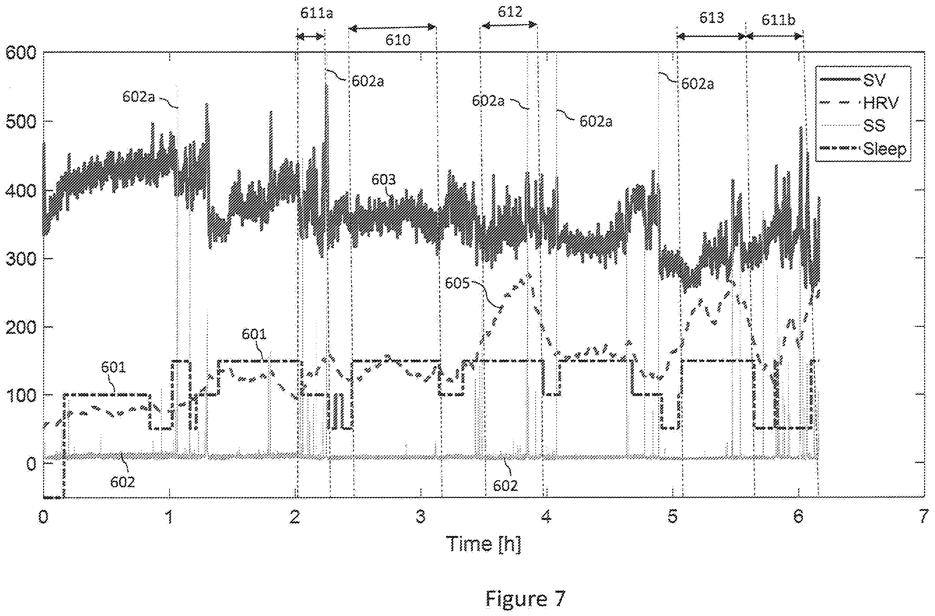

Figure 7 illustrates another example of a plot showing signals obtained by processing a single BCG signal according to the invention, representing a recording over a whole night with some 6 hours of sleep. This plot shows output with a step-like sleep stage signal (601), a solid line stroke volume signal (603), a dotted line heart rate variability signal (605), and signal strength signal (602). The Y-axis represents an arbitrary scale that facilitates clear distinction between various signals for illustration purposes. The subject is first awake, but falls asleep at about 0.15-0.20 hours, as indicated with the sleep curve (601) rising from value -50, that indicates being awake before sleeping, to value 100 that indicates shallow sleep. Period 611a just after two hours of recording provides an example of a period during which the SS signal has some high peaks, which indicates that the subject is moving, and sleep is shallow. Another period 611b with lots of movement can be found around 6 hours of recording. This period represents mainly REM sleep, with the sleep stage signal 601 value 50. During such periods of movement there is lots of variation in the stroke volume signal, but the SS signal values indicate that these variations are not likely to be caused by abnormal heart beats, but movement of the subject.

The period 610 is an exemplary period of normal, peaceful deep sleep with no detected arrhythmia, similar to period 610 of the figure 6. Stroke volume signal (603) indicates good and healthy stoke volumes during the normal sleep and heart beat period 610. Heart rate variation signal (605) has higher values than for example during the first shallow sleep period between 0 and 1 hours. This is likely due to recovery already occurred during the sleep.

The figure 7 also shows periods of persistent or continuous arrhythmia (612, 613). Arrhythmia may be identified from a combination of little or no movement indicated by the signal strength signal (602), but increased HRV signal (605), in other words higher heart rate variability HRV signal (605) values than during normal sleep period (610), and/or abnormal beat-to-beat times. Heart beatTB2B and/or HRV signals obtained during this a period of arrhythmia may be analyzed in more detail to recognize the type of arrhythmia.

The period 613 also comprises mainly normal signal strength (SS) levels that indicate restful, deep sleep with only a couple of signal strength signal (602) peaks, but substantial variations in the HRV signal (605). An drop in the overall stroke volume signal (603) level compared for example with the preceding sleep periods also indicates occurrence of an abnormal heart beat situation, indicating that chambers of the heart cannot fully utilize their normal capacity during this period. Stroke volume (603) is somewhat lower than during the normal heart beat period (610), and the HRV signal (605) is clearly higher than during the normal heart beat period (610). These are clear indicators of arrhythmia.

While the obtained BCG signal received from a single accelerometer is quite weak, it is possible that noise from the environment, for example nearby traffic or mechanical devices such as pumps, causes acceleration signals to be detected that could be erroneously interpreted as BCG signals of the subject. However, it is normally possible to distinguish such noise-origin signals from signals obtained from a real subject. Typically, a signal caused by the environment has lower signal strength than a signal obtained from a subject. Thus, if the signal strength is very weak, an abnormal beat-to-beat time, stroke volume or too high HRV may be disregarded as being likely caused by noise.

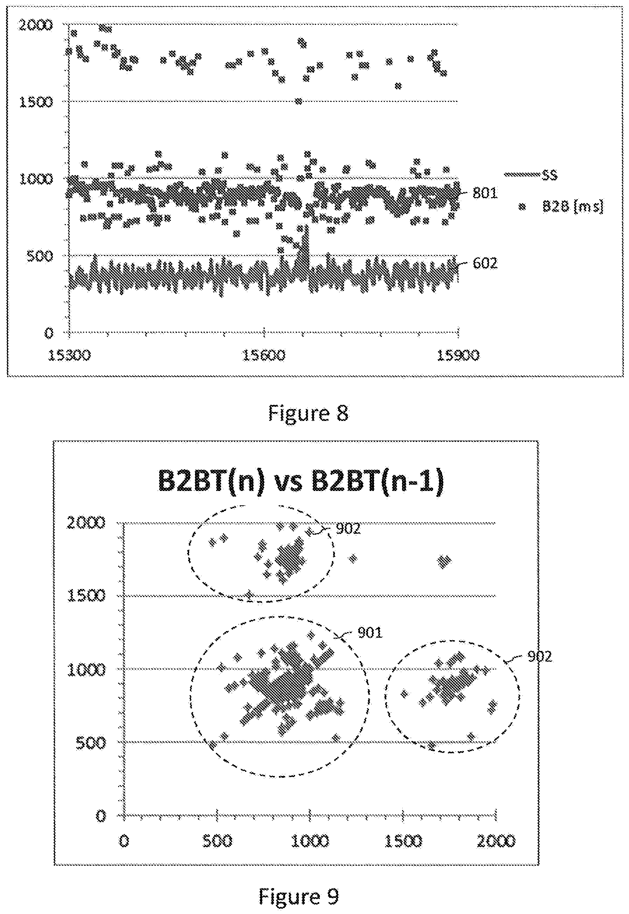

Figure 8 illustrates a plot of signal strength and beat-to-beat times during an exemplary detected arrhythmia during a period of a few minutes. Signal strength (602) illustrated by a solid line remains relatively stable in a level that indicates no movement nor weak signal level that could be interpreted as being mere noise. Thus, the plot may be expected to represent actual obtained beat-to-beat times of a subject. However, beat-to-beat times (801) marked with dots vary significantly, and form clear groups. Majority of the beat-to-beat times (801) have a value in the range of 500-1200 ms, but another clearly visible group of beat-to-beat times can be seen approximately in the range between 1500 and 2000 ms. Such pattern of normal and extended beat-to-beat times (801) indicates occurrence of bradycardia, which means missing or delayed heart beats.

Figure 9 illustrates an exemplary recurrence plot, known as Poincaré or Lorenz plot, over the same time period illustrated in the plot of the figure 8, that shows another view to the differences between consecutive beat-to-beat times. The plot compares each beat-to-beat time B2BT(n) to the preceding beat-to-beat time B2BT(n-1). In this plot, normal beat-to-beat times form a first group (901), while two relatively symmetrically placed side groups (902) are shown that indicate extended, roughly double, beat-to-beat times. Based on the indicated variation of beat-to-beat times, the type of arrhythmia may be recognized as bradycardia.

It is apparent to a person skilled in the art that as technology advanced, the basic idea of the invention can be implemented in various ways. The invention and its embodiments are therefore not restricted to the above examples, but they may vary within the scope of the claims.

## Claims

1. A system providing temporal information on a subject, the system comprising:
- an accelerometer configured to obtain (200) a ballistocardiographic signal of a subject;
- processing means configured to process the ballistocardiographic signal to obtain (201) at least a TB2B (beat-to-beat time) signal and (202) an SV (stroke volume) signal, wherein the processing means comprises:
- a first filter (211) configured to produce a filtered TB2B signal by filtering the TB2B signal to remove erroneous TB2B signal peaks not caused by heart beats, wherein the first filter is configured:
- to calculate (411) an average TB2B signal strength (602) over a first time period;
- to define (412) an allowed variation range for the TB2B signal strength (602); and
- to remove (413) any peaks from the TB2B signal that do not fit within the allowed variation range for producing the filtered TB2B signal;
- a second filter (212) configured to produce a filtered SV signal (603) by filtering the SV signal to remove erroneous SV signal peaks not caused by heart beats, wherein the second filter is configured:
- to calculate (511) an average root mean square signal strength for the SV over a moving time window;
- to define (512) an allowed SV signal strength variation range for SV signal strength; and
- to remove (513) any SV signal peaks that do not fit within the allowed SV signal strength variation range;
- calculation means configured to calculate (231) a true HR signal from the filtered TB2B signal, wherein the true HR signal is substantially free from errors caused by erroneous peaks not caused by heart beats;
- calculation means configured to calculate (222) a stroke volume variability, SVV, signal from the filtered SV signal (603);
- a third filter configured to filter (221) the true HR signal to detect abnormal heart beats;
wherein the processing means is configured to filter the filtered SV signal (603) and the SVV signal for detecting changes in variation of the stroke volume and using such changes in variation of the stoke volume as additional parameters facilitating separation (221) of abnormal heart beats from normal heart beats, and if abnormal heartbeats are detected, the processing means is further configured to identify (241) type of arrhythmia related to the abnormal heart beats and wherein an output unit is configured to output (251) temporal information on occurrence and type of the identified arrhythmia and temporal information on the detected variations of stroke volume,
wherein the processing means is further configured:
- to obtain information on occurrence of at least one of the erroneous TB2B signal peaks and erroneous SV signal values in connection to filtering (211) the TB2B signal and filtering (212) the SV signal;
- to obtain (204) a signal strength (SS) signal (602) by calculating an average root mean square of the strength of the received ballistocardiographic signal; and
- to use the SS signal (602) for discriminating whether the received signal represents actual detected BCG signal, background noise, or movement of the subject, wherein a SS signal (602) within a predefined signal strength interval is deemed to represent actual BCG measurement result, a SS signal (602) weaker than the predefined signal strength interval is deemed to represent noise, and a SS signal (602) stronger than the predefined signal strength interval is deemed to represent movement of the subject, and in that
- the output unit is further configured to provide (251) a temporal indication of movement of the subject on basis of the information on occurrence of at least one of the erroneous TB2B signal peaks and the erroneous SV signal values.

2. The system according to claim 1, wherein the processing means is further configured:
- to calculate (203) a respiration rate (RR) signal;
- to calculate (231) a HFHRV (high frequency heart rate variability) signal and at least one of a LFHRV (low frequency heart rate variation) signal and a VLFHRV (very low frequency heart rate variation) signal from the filtered TB2B signal;
- to analyze the SV signal, the RR signal and at least one of the LFHRV and VLFHRV signals for determining (242) occurrence of a sleep disorder, and if one or more instances of sleep disorder is determined to have occurred, to identify the type of sleep disorder;
- to output (251) temporal information on the occurrence and identified type of sleep disorder in combination with the temporal information on arrhythmia;
- to determine (243) temporal information on at least one of stress, nocturnal recovery and sleep phase by analyzing at least the RR signal, the HFHRV signal and the LFHRV signal; and
- to output (251) temporal information on the determined at least one of stress, recovery and sleep phase and sleep disorder in combination with the temporal information on arrhythmia.

3. The system according to any of claims 1 to 2, wherein the filtering the true HR signal comprises:
- defining a normal beat-to-beat time during a third time period;
- specifying a time window corresponding to normal variation of the beat-to-beat times during the third time period; and
- identifying any beat-to-beat times shorter or longer than the time window as abnormal heart beats indicating arrhythmia.

4. The system according to claim 3, wherein the time window is defined dynamically based on historical HRV data of the subject.

5. The system according to any of claims 1 to 4, wherein the accelerometer is configured to obtain the ballistocardiographic signal of the subject by measuring acceleration in the longitudinal direction of the subject.

6. A computer program product configured to perform a method of providing temporal information on a subject, which, when executed by a computing device or system (102, 104) cause the computing system or device (102, 104) to perform the steps of:
- receiving (200) a ballistocardiographic signal of a subject;
- processing the ballistocardiographic signal to obtain (201) at least a beat-to-beat time (TB2B) signal and (202) a stroke volume (SV) signal;
- producing a filtered TB2B signal by filtering (211) the TB2B signal to remove erroneous values not caused by heart beats, wherein said filtering the TB2B signal comprises:
- calculating (411) an average TB2B signal strength over a first time period;
- defining (412) an allowed variation range for the TB2B signal strength; and
- removing (413) any peaks from the TB2B signal that do not fit within the allowed variation range for producing the filtered TB2B signal;
- producing a filtered SV signal (603) by filtering (212) the SV signal to remove erroneous SV values not caused by heart beats, wherein said filtering the SV signal comprises:
- calculating (511) an average root mean square signal strength for the SV over a moving time window;
- defining (512) an allowed SV signal strength variation range for SV signal strength; and
- removing (513) any SV signal peaks that do not fit within the allowed SV signal strength variation range;
- calculating (231) a true HR signal from the filtered TB2B signal, wherein the true HR signal is substantially free from errors caused by erroneous peaks not caused by heart beats;
- calculating (222) a stroke volume variability (SVV) signal from the filtered SV signal (603);
- filtering the true HR signal to detect abnormal heart beats and filtering at least one of the filtered SV signal (603) and the SVV signal for detecting changes in variation of the stroke volume and using such changes in variation of the stroke volume as additional parameters facilitating separation (221) of abnormal heart beats from them from normal heart beats; and
- if abnormal heart beats are detected, further identifying (241) type of arrhythmia related to the detected abnormal heart beats, and
- outputting (251) temporal information on occurrence and type of the identified arrhythmia and temporal information on the detected changes in variation of stroke volume,
wherein the steps further comprise:
- in connection to filtering (211) the TB2B signal and filtering (212) the SV signal, obtaining information on occurrence of at least one of the erroneous TB2B signal peaks and erroneous SV signal values;
- obtaining (204) a signal strength (SS) signal (602) by calculating an average root mean square of the strength of the received ballistocardiographic signal;
- using the SS signal (602) for discriminating whether the received signal represents actual detected BCG signal, background noise, or movement of the subject, wherein a SS signal (602) within a predefined signal strength interval is deemed to represent actual BCG measurement result, a SS signal (602) weaker than the predefined signal strength interval is deemed to represent noise, and a SS signal (602) stronger than the predefined signal strength interval is deemed to represent movement of the subject; and
- outputting (251) temporal indication of movement of the subject on basis of the information on occurrence of at least one of the erroneous TB2B signal peaks and the erroneous SV signal values.

7. The computer program product according to claim 7, further causing the computing system or device to perform the steps of:
- calculating (203) a respiration rate (RR) signal;
- calculating (231) a HFHRV (high frequency heart rate variability) signal and at least one of a LFHRV (low frequency heart rate variation) signal, a VLFHRV (very low frequency heart rate variation) signal from the filtered TB2B signal;
- analyzing the filtered SV signal (603), the RR signal and at least one of the LFHRV and VLFHRV signals for determining (242) occurrence of sleep disorder, and if one or more instances of sleep disorder is determined to have occurred, identifying the type of sleep disorder and outputting (251) temporal information on the occurrence and the identified type of sleep disorder in combination with the temporal information on arrhythmia; and
- analyzing at least the RR signal, the HFHRV signal and the LFHRV signal for determining (243) temporal information of at least one of stress, recovery and sleep phase and outputting (251) the respective temporal information on the determined at least one of stress, recovery and sleep phase in combination with the temporal information on arrhythmia.

8. The computer program product according to claim 6 or 7, wherein the filtering the true HR signal comprises:
- defining a normal beat-to-beat time during a third time period;
- specifying a time window corresponding to normal variation of the beat-to-beat times during the third time period; and
- identifying any beat-to-beat times shorter or longer than the time window as abnormal heart beats.

9. The computer program product according to claim 8, wherein the time window is defined dynamically based on historical HRV data of the subject.

10. The computer program product according to any of claims 6 to 9, further causing the computing system or device to perform the steps of:
Obtaining (200) the ballistocardiographic signal of the subject from an accelerometer measuring acceleration in the longitudinal direction of the subject.

## Patentansprüche

1. System, das zeitliche Informationen über einen Patienten bereitstellt, wobei das System umfasst:
- einen Beschleunigungsmesser, der dazu ausgelegt ist, ein ballistokardiografisches Signal eines Patienten zu erhalten (200);
- ein Verarbeitungsmittel, das dazu ausgelegt ist, das ballistokardiografische Signal zu verarbeiten, um (201) mindestens ein TB2B-(Schlag-zu-Schlag-Zeit-)Signal und (202) ein SV-(Schlagvolumen-)Signal zu erhalten, wobei das Verarbeitungsmittel umfasst:
- ein erstes Filter (211), das ausgelegt ist zum Erzeugen eines gefilterten TB2B-Signals durch Filtern des TB2B-Signals zur Eliminierung von fehlerhaften nicht von Herzschlägen verursachten TB2B-Signalspitzen, wobei das erstes Filter ausgelegt ist zum:
- Berechnen (411) einer durchschnittlichen TB2B-Signalstärke (602) über einen ersten Zeitraum;
- Definieren (412) eines zulässigen Variationsbereichs für die TB2B-Signalstärke (602); und
- Eliminieren (413) aller Spitzen aus dem TB2B-Signal, die nicht in den zulässigen Variationsbereich passen, zum Erzeugen des gefilterten TB2B-Signals;
- ein zweites Filter (212), das ausgelegt ist zum Erzeugen eines gefilterten SV-Signals (603) durch Filtern des SV-Signals zur Eliminierung von fehlerhaften nicht von Herzschlägen verursachten SV-Signalspitzen, wobei das zweite Filter ausgelegt ist zum:
- Berechnen (511) einer quadratisch gemittelten durchschnittlichen Signalstärke für das SV über ein bewegliches Zeitfenster;
- Definieren (512) eines zulässigen SV-Signalstärkenvariationsbereichs für die SV-Signalstärke; und
- Eliminieren (513) aller SV-Signalspitzen, die nicht in den zulässigen SV-Signalstärkenvariationsbereich passen;
- ein Berechnungsmittel, das ausgelegt ist zum Berechnen (231) eines wahren HR-Signals aus dem gefilterten TB2B-Signal, wobei das wahre HR-Signal im Wesentlichen frei von Fehlern ist, die durch nicht-herzschlagbedingte fehlerhafte Spitzen verursacht werden;
- ein Berechnungsmittel, das ausgelegt ist zum Berechnen (222) eines Schlagvolumenvariabilitäts-SVV-Signals aus dem gefilterten SV-Signal (603);
- ein drittes Filter, das ausgelegt ist zum Filtern (221) des wahren HR-Signals zur Detektion von anormalen Herzschlägen;
wobei das Verarbeitungsmittel ausgelegt ist zum Filtern des gefilterten SV-Signals (603) und des SVV-Signals zur Detektion von Änderungen der Variation des Schlagvolumens und zur Verwendung dieser Änderungen der Variation des Schlagvolumens als zusätzliche Parameter, die eine Trennung (221) der anomalen Herzschläge von normalen Herzschlägen begünstigen, und, falls anomale Herzschläge detektiert werden, das Verarbeitungsmittel ferner ausgelegt ist zum Identifizieren (241) der auf die anomalen Herzschläge bezogenen Art von Herzrhythmusstörung, und wobei eine Ausgabeeinheit ausgelegt ist zum Ausgeben (251) von zeitlichen Informationen über das Auftreten und die Art der identifizierten Herzrhythmusstörung und zeitlichen Informationen über die detektierten Variationen des Schlagvolumens,
wobei das Verarbeitungsmittel ferner ausgelegt ist zum:
- Erhalten von Informationen über das Auftreten von fehlerhaften TB2B-Signalspitzen und/oder fehlerhafte SV-Signalwerten in Verbindung mit dem Filtern (211) des TB2B-Signals und dem Filtern (212) des SV-Signals;
- Erhalten (204) eines Signalstärke-(SS)-Signals (602) durch Berechnen eines quadratischen gemittelten Durchschnitts der Stärke des empfangenen ballistokardiografischen Signals; und
- Verwenden des SS-Signals (602) zur Unterscheidung, ob das empfangene Signal ein tatsächliches detektiertes BKG-Signal, Hintergrundrauschen oder eine Bewegung des Patienten darstellt, wobei davon ausgegangen wird, dass ein SS-Signal (602) innerhalb eines vordefinierten Signalstärkenbereichs das tatsächliche BKG-Messergebnis darstellt, ein SS-Signal (602), das schwächer als der vordefinierte Signalstärkenbereich ist, ein Rauschen darstellt, und ein SS-Signal (602), das stärker als der vordefinierte Signalstärkenbereich ist, eine Bewegung des Patienten darstellt, und dadurch, dass
- die Ausgabeeinheit ferner ausgelegt ist zum Bereitstellen (251) einer zeitlichen Anzeige der Bewegung des Patienten auf Grundlage der Informationen über das Auftreten der fehlerhaften TB2B-Signalspitzen und/oder der fehlerhaften SV-Signalwerte.

2. System nach Anspruch 1, wobei das Verarbeitungsmittel ferner ausgelegt ist zum:
- Berechnen (203) eines Atemfrequenz-(RR)-Signals;
- Berechnen (231) eines HFHRV-(hochfrequenten Herzratenvariabilitäts-)Signals sowie eines LFHRV-(niederfrequenten Herzratenvariabilitäts-)Signals und/oder VLFHRV-(sehr niederfrequenten Herzratenvariabilitäts-)Signals aus dem gefilterten TB2B-Signal;
- Analysieren des SV-Signals, des RR-Signals sowie des LFHRV- und/oder VLFHRV-Signals zum Bestimmen (242) des Auftretens einer Schlafstörung und, falls bestimmt wird, dass eine oder mehrere Instanzen von Schlafstörung aufgetreten sind, Identifizieren der Art von Schlafstörung;
- Ausgeben (251) von zeitlichen Informationen über das Auftreten und die identifizierte Art von Schlafstörung in Kombination mit den zeitlichen Informationen über die Herzrhythmusstörung;
- Bestimmen (243) von zeitlichen Informationen über mindestens eines der Elemente Stress, nächtliche Erholung und Schlafphase durch Analysieren mindestens des RR-Signals, des HFHRV-Signals und des LFHRV-Signals; und
- Ausgeben (251) von zeitlichen Informationen über das bestimmte mindestens eine Element Stress, Erholung und Schlafphase sowie Schlafstörung in Kombination mit den zeitlichen Informationen über die Herzrhythmusstörung.

3. System nach einem der Ansprüche 1 bis 2, wobei das Filtern des wahren HR-Signals umfasst:
- Definieren einer normalen Schlag-zu-Schlag-Zeit während eines dritten Zeitraums;
- Festlegen eines Zeitfensters, das der normalen Variation der Schlag-zu-Schlag-Zeiten während des dritten Zeitraums entspricht; und
- Identifizieren aller Schlag-zu-Schlag-Zeiten, die kürzer oder länger als das Zeitfenster sind, als anomale Herzschläge, die eine Herzrhythmusstörung anzeigen.

4. System nach Anspruch 3, wobei das Zeitfenster auf Grundlage von historischen HRV-Daten des Patienten dynamisch definiert wird.

5. System nach einem der Ansprüche 1 bis 4, wobei der Beschleunigungsmesser dazu ausgelegt ist, das ballistokardiografische Signal des Patienten dadurch zu erhalten, dass die Beschleunigung in der Längsrichtung des Patienten gemessen wird.

6. Computerprogrammprodukt, das dazu ausgelegt ist, ein Verfahren zur Bereitstellung von zeitlichen Informationen über einen Patienten auszuführen, welches, wenn es auf einer Rechenvorrichtung oder einem Rechensystem (102, 104) ausgeführt wird, das Rechensystem oder die Rechenvorrichtung (102, 104) dazu veranlasst, die folgenden Schritte auszuführen:
- Empfangen (200) eines ballistokardiografischen Signals eines Patienten;
- Verarbeiten des ballistokardiografischen Signals zum Erhalten (201) mindestens eines Schlag-zu-Schlag-Zeit-(TB2B)-Signals und (202) eines Schlagvolumen-(SV)-Signals;
- Erzeugen eines gefilterten TB2B-Signals durch Filtern (211) des TB2B-Signals zum Eliminieren von fehlerhaften nicht von Herzschlägen verursachten Werten, wobei das Filtern des TB2B-Signals umfasst:
- Berechnen (411) einer durchschnittlichen TB2B-Signalstärke über einen ersten Zeitraum;
- Definieren (412) eines zulässigen Variationsbereichs für die TB2B-Signalstärke; und
- Eliminieren (413) aller Spitzen aus dem TB2B-Signal, die nicht in den zulässigen Variationsbereich passen, zum Erzeugen des gefilterten TB2B-Signals;
- Erzeugen eines gefilterten SV-Signals (603) durch Filtern (212) des SV-Signals zum Eliminieren von fehlerhaften nicht von Herzschlägen verursachten SV-Werten, wobei das Filtern des SV-Signals umfasst:
- Berechnen (511) einer quadratisch gemittelten durchschnittlichen Signalstärke für das SV über ein bewegliches Zeitfenster;
- Definieren (512) eines zulässigen SV-Signalstärkenvariationsbereichs für die SV-Signalstärke; und
- Eliminieren (513) aller SV-Signalspitzen, die nicht in den zulässigen SV-Signalstärkenvariationsbereich passen;
- Berechnen (231) eines wahres HR-Signals aus dem gefilterten TB2B-Signal, wobei das wahre HR-Signal im Wesentlichen frei von Fehlern ist, die durch nicht-herzschlagbedingte fehlerhafte Spitzen verursacht werden;
- Berechnen (222) eines Schlagvolumenvariabilitäts-(SVV)-Signals aus dem gefilterten SV-Signal (603);
- Filtern des wahren HR-Signals zum Detektieren von anomalen Herzschlägen und Filtern des gefilterten SV-Signals (603) und/oder des SVV-Signals zum Detektieren von Änderungen der Variation des Schlagvolumens und Verwenden dieser Änderungen der Variation des Schlagvolumens als zusätzliche Parameter, die die Trennung (221) der anomalen Herzschläge von normalen Herzschlägen begünstigen; und
- falls anomale Herzschläge detektiert werden, ferner das Identifizieren (242) der auf die detektierten anomalen Herzschläge bezogenen Art von Herzrhythmusstörung, und
- Ausgeben (251) von zeitlichen Informationen über das Auftreten und die Art der identifizierten Herzrhythmusstörung und von zeitlichen Informationen über die detektierten Änderungen der Variation des Schlagvolumens,
wobei die Schritte ferner umfassen:
- in Verbindung mit dem Filtern (211) des TB2B-Signals und Filtern (212) des SV-Signals, Erhalten von Informationen über das Auftreten der fehlerhaften TB2B-Signalspitzen und/oder fehlerhaften SV-Signalwerte;
- Erhalten (204) eines Signalstärke-(SS)-Signals (602) durch Berechnen eines quadratisch gemittelten Durchschnitts der Stärke des empfangenen ballistokardiografischen Signals;
- Verwenden des SS-Signals (602) zur Unterscheidung, ob das empfangene Signal ein tatsächliches detektiertes BKG-Signal, Hintergrundrauschen oder eine Bewegung des Patienten darstellt, wobei davon ausgegangen wird, dass ein SS-Signal (602) innerhalb eines vordefinierten Signalstärkenbereichs das tatsächliche BKG-Messergebnis darstellt, ein SS-Signal (602), das schwächer als der vordefinierte Signalstärkenbereich ist, ein Rauschen darstellt, und ein SS-Signal (602), das stärker als der vordefinierte Signalstärkenbereich ist, eine Bewegung des Patienten darstellt; und
- Ausgeben (251) einer zeitlichen Anzeige der Bewegung des Patienten auf Grundlage der Informationen über das Auftreten der fehlerhaften TB2B-Signalspitzen und/oder der fehlerhaften SV-Signalwerte bereitzustellen.

7. Computerprogrammprodukt nach Anspruch 7, das das Rechensystem oder die Rechenvorrichtung ferner zum Ausführen der folgenden Schritte veranlasst:
- Berechnen (203) eines Atemfrequenz-(RR)-Signals;
- Berechnen (231) eines HFHRV-(hochfrequenten Herzratenvariabilitäts-)Signals sowie eines LFHRV-(niederfrequenten Herzratenvariabilitäts-)Signals und/oder VLFHRV-(sehr niederfrequenten Herzratenvariabilitäts-)Signals aus dem gefilterten TB2B-Signal;
- Analysieren des gefilterten SV-Signals (603), des RR-Signals sowie des LFHRV- und/oder VLFHRV-Signals zum Bestimmen (242) des Auftretens einer Schlafstörung und, falls bestimmt wird, dass eine oder mehrere Instanzen von Schlafstörung aufgetreten sind, Identifizieren der Art von Schlafstörung und Ausgeben (251) von zeitlichen Informationen über das Auftreten und die identifizierte Art von Schlafstörung in Kombination mit den zeitlichen Informationen über die Herzrhythmusstörung; und
- Analysieren mindestens des RR-Signals, des HFHRV-Signals und des LFHRV-Signals zum Bestimmen (243) von zeitlichen Informationen über das mindestens eine Element Stress, Erholung und Schlafphase, und Ausgeben (251) der entsprechenden zeitlichen Informationen über das bestimmte mindestens eine Element Stress, Erholung und Schlafphase in Kombination mit den zeitlichen Informationen über die Herzrhythmusstörung.

8. Computerprogrammprodukt nach Anspruch 6 oder 7, wobei das Filtern des wahren HR-Signals umfasst:
- Definieren einer normalen Schlag-zu-Schlag-Zeit während eines dritten Zeitraums;
- Festlegen eines Zeitfensters, das der normalen Variation der Schlag-zu-Schlag-Zeiten während des dritten Zeitraums entspricht; und
- Identifizieren aller Schlag-zu-Schlag-Zeiten, die kürzer oder länger als das Zeitfenster sind, als anomale Herzschläge.

9. Computerprogrammprodukt nach Anspruch 8, wobei das Zeitfenster auf Grundlage von historischen HRV-Daten des Patienten dynamisch definiert wird.

10. Computerprogrammprodukt nach einem der Ansprüche 6 bis 9, das das Rechensystem oder die Rechenvorrichtung ferner zum Ausführen der folgenden Schritte veranlasst:
Erhalten (200) des ballistokardiografischen Signals des Patienten von einem Beschleunigungsmesser, der die Beschleunigung in der Längsrichtung des Patienten misst.

## Revendications

1. Système fournissant des informations temporelles sur un sujet, ledit système comprenant :
- un accéléromètre configuré pour obtenir (200) un signal ballistocardiographique d'un sujet;
- un moyen de traitement configuré pour traiter le signal ballistocardiographique afin d'obtenir (201) au moins un signal TB2B (temps entre deux battements) et (202) un signal SV (volume systolique), ledit moyen de traitement comprenant :
- un premier filtre (211) configuré pour produire un signal TB2B filtré en filtrant le signal TB2B afin d'éliminer des pics de signal TB2B erronés non causés par des battements cardiaques, ledit premier filtre étant configuré pour :
- calculer (411) une intensité de signal TB2B moyenne (602) sur une première période de temps;
- définir (412) une plage de variation autorisée pour l'intensité de signal TB2B (602); et
- supprimer (413) tous les pics du signal TB2B qui ne sont pas comprises dans la plage de variation autorisée afin de produire le signal TB2B filtré;
- un deuxième filtre (212) configuré pour produire un signal SV filtré (603) en filtrant le signal SV afin d'éliminer des pics de signal SV erronés non causés par des battements cardiaques, ledit deuxième filtre étant configuré pour :
- calculer (511) une intensité de signal quadratique moyenne pour le SV sur une fenêtre temporelle mobile;
- définir (512) une plage de variation d'intensité de signal SV autorisée pour l'intensité de signal SV; et
- supprimer (513) tous les pics de signal SV qui ne sont pas comprises dans la plage de variation autorisée de l'intensité de signal SV;
- un moyen de calcul configuré pour calculer (231) un signal HR vrai à partir du signal TB2B filtré, ledit signal HR vrai étant essentiellement exempt d'erreurs causées par des pics erronés non causés par des battements cardiaques;
- un moyen de calcul configuré pour calculer (222) un signal de variabilité de volume systolique, SVV, à partir du signal SV filtré (603),
- un troisième filtre configuré pour filtrer (221) le signal HR vrai afin de détecter des battements cardiaques anormaux;
dans lequel le moyen de traitement est configuré pour filtrer le signal SV filtré (603) et le signal SVV afin de détecter des changements de variation du volume systolique et d'utiliser lesdits changements de variation du volume systolique comme paramètres supplémentaires facilitant la séparation (221) des battements cardiaques anormaux des battements cardiaques normaux, et, si des battements cardiaques anormaux sont détectés, le moyen de traitement est également configurés pour identifier (241) le type d'arythmie lié aux battements cardiaques anormaux, et dans lequel une unité de sortie est configurée pour fournir (251) des informations temporelles sur l'apparition et le type de l'arythmie identifiée et des informations temporelles sur les variations détectées du volume systolique,
dans lequel le moyen de traitement est également configuré :
- pour obtenir des informations sur l'apparition d'au moins l'un parmi les pics de signal T2B2 erronés et les valeurs de signal SV erronées en liaison avec le filtrage (211) du signal TB2B et le filtrage (212) du signal SV;
- obtenir (204) un signal d'intensité de signal (SS) (602) en calculant une moyenne quadratique de l'intensité du signal ballistocardiographique reçu; et
- pour utiliser le signal SS (602) afin de distinguer si le signal reçu représente un signal BCG réel détecté, un bruit de fond ou un mouvement du sujet, dans lequel un signal SS (602) compris dans un intervalle d'intensité de signal prédéfini est considéré comme représentant un résultat de mesure BCG réel, un signal SS (602) plus faible que l'intervalle d'intensité de signal prédéfini est considéré comme représentant du bruit, et un signal SS (602) plus fort que l'intervalle d'intensité de signal prédéfini est considéré comme représentant un mouvement du sujet, et en ce que
- l'unité de sortie est également configurée pour fournir (251) une indication temporelle du mouvement du sujet sur la base des informations sur l'apparition d'au moins l'un parmi les pics de signal TB2B erronés et les valeurs de signal SV erronées.

2. Système selon la revendication 1, ledit moyen de traitement étant également configuré :
- pour calculer (203) un signal de fréquence respiratoire (RR);
- pour calculer (231) un signal HFHRV (variabilité de la fréquence cardiaque en haute fréquence) et au moins l'un parmi un signal LFHRV (variabilité de la fréquence cardiaque en basse fréquence) et un signal VLFHRV (variabilité de la fréquence cardiaque en très basse fréquence) à partir du signal TB2B filtré;
- pour analyser le signal SV, le signal RR et au moins l'un des signaux LFHRV et VLFHRV afin de déterminer (242) l'apparition d'un trouble de sommeil et, s'il est déterminé qu'il y a eu un ou plusieurs cas d'apparition de trouble de sommeil, pour identifier le type de trouble de sommeil;
- pour fournir (251) des informations temporelles sur l'apparition et le type identifié du trouble de sommeil en combinaison avec les informations temporelles sur l'arythmie;
- pour déterminer (243) des informations temporelles sur au moins l'un parmi le stress, la récupération nocturne et la phase de sommeil en analysant au moins le signal RR, le signal HFHRV et le signal LFHRV; et
- fournir (251) des informations temporelles sur ledit au moins l'un déterminé parmi le stress, la récupération et la phase de sommeil et le trouble de sommeil en combinaison avec les informations temporelles sur l'arythmie.

3. Système selon l'une des revendications 1 à 2, dans lequel le filtrage du signal HR vrai comprend :
- définir un temps entre deux battements normal pendant une troisième période;
- spécifier une fenêtre temporelle correspondant à la variation normale des temps entre deux battements pendant la troisième période; et
- identifier tous les temps entre deux battements plus courts ou plus longs que la fenêtre temporelle comme battements cardiaques anormaux indiquant une arythmie.

4. Système selon la revendication 3, dans lequel la fenêtre temporelle est définie de manière dynamique sur la base des données HRV historiques du sujet.

5. Système selon l'une des revendications 1 à 4, dans lequel l'accéléromètre est configuré pour obtenir le signal ballistocardiographique du sujet en mesurant l'accélération dans la direction longitudinale du sujet.

6. Produit de programme informatique configuré pour exécuter un procédé pour fournir des informations temporelles sur un sujet, qui, lorsqu'il est exécuté par un dispositif ou système informatique (102, 104), amène le système ou dispositif informatique (102, 104) à exécuter les étapes consistant à :
- recevoir (200) un signal ballistocardiographique d'un sujet;
- traiter un signal ballistocardiographique pour obtenir (201) au moins un signal de temps entre deux battements (TBSB) et (202) un signal de volume systolique (SV);
- produire un signal TB2B filtré en filtrant (211) le signal TB2B pour supprimer les valeurs erronées non causées par des battements cardiaques, ledit filtrage du signal TB2B comprenant les étapes consistant à :
- calculer (411) une intensité de signal TB2B moyenne sur une première période de temps;
- définir (412) une plage de variation autorisée pour l'intensité de signal TB2B; et
- supprimer (413) tous les pics du signal TB2B qui ne sont pas comprises dans la plage de variation autorisée afin de produire le signal TB2B filtré;
produire un signal SV filtré (603) en filtrant (212) le signal SV pour supprimer les valeurs SV erronées non causées par des battements cardiaques, ledit filtrage du signal SV comprenant les étapes consistant à :
- calculer (511) une intensité de signal quadratique moyenne pour le SV sur une fenêtre temporelle mobile;
- définir (512) une plage de variation d'intensité de signal SV autorisée pour l'intensité de signal SV; et
- supprimer (513) tous les pics de signal SV qui ne sont pas comprises dans la plage de variation autorisée de l'intensité de signal SV;
calculer (231) un signal HR vrai à partir du signal T2B2 filtré, ledit signal HR vrai étant essentiellement exempt d'erreurs causées par des pics erronés non causés par des battements cardiaques;
- calculer (222) un signal de variabilité de volume systolique (SVV) à partir du signal SV filtré (603);
- filtrer le signal HR vrai pour détecter des battements cardiaques anormaux et filtrer au moins l'un parmi le signal SV filtré (603) et le signal SVV afin de détecter des changements de variation du volume systolique et d'utiliser lesdits changements de variation du volume systolique comme paramètres supplémentaires facilitant la séparation (221) des battements cardiaques anormaux des battements cardiaques normaux; et
- si des battements cardiaques anormaux sont détectés, identifier (242) également le type d'arythmie lié aux battements cardiaques anormaux détectés, et
- fournir (251) des informations temporelles sur l'apparition et le type de l'arythmie identifiée et des informations temporelles sur les changements de variation détectés du volume systolique,
dans lequel les étapes comprennent également les étapes consistant à :
- en relation avec le filtrage (211) du signal TB2B et le filtrage (212) du signal SV, obtenir des informations sur l'apparition d'au moins l'un parmi les pics de signal TB2B erronés et les valeurs de signal SV erronées;
- obtenir (204) un signal d'intensité de signal (SS) (602) en calculant une moyenne quadratique de l'intensité du signal ballistocardiographique reçu;
- utiliser le signal SS (602) afin de distinguer si le signal reçu représente un signal BCG réel détecté, un bruit de fond ou un mouvement du sujet, dans lequel un signal SS (602) compris dans un intervalle d'intensité de signal prédéfini est considéré comme représentant un résultat de mesure BCG réel, un signal SS (602) plus faible que l'intervalle d'intensité de signal prédéfini est considéré comme représentant du bruit, et un signal SS (602) plus fort que l'intervalle d'intensité de signal prédéfini est considéré comme représentant un mouvement du sujet; et
- fournir (251) une indication temporelle du mouvement du sujet sur la base des informations sur l'apparition d'au moins l'un parmi les pics de signal TB2B erronés et les valeurs de signal SV erronées.

7. Produit de programme informatique selon la revendication 7, qui amène le système ou dispositif informatique également à effectuer les étapes consistant à :
- calculer (203) un signal de fréquence respiratoire (RR);
- calculer (231) un signal HFHRV (variabilité de la fréquence cardiaque en haute fréquence) et au moins l'un parmi un signal LFHRV (variabilité de la fréquence cardiaque en basse fréquence) et un signal VLFHRV (variabilité de la fréquence cardiaque en très basse fréquence) à partir du signal TB2B filtré;
- analyser le signal SV filtré (603), le signal RR et au moins l'un des signaux LFHRV et VLFHRV afin de déterminer (242) l'apparition d'un trouble de sommeil et, s'il est déterminé qu'il y a eu un ou plusieurs cas d'apparition de trouble de sommeil, identifier le type de trouble de sommeil et fournir (251) des informations temporelles sur l'apparition et le type identifié du trouble de sommeil en combinaison avec les informations temporelles sur l'arythmie; et
- analyser au moins le signal RR, le signal HFHRV et le signal LFHRV pour déterminer (243) des informations temporelles sur au moins l'un parmi le stress, la récupération et la phase de sommeil, et fournir (251) les informations temporelles respectives sur ledit au moins l'un déterminé parmi le stress, la récupération et la phase de sommeil en combinaison avec les informations temporelles sur l'arythmie.

8. Produit de programme informatique selon la revendication 6 ou 7, dans lequel le filtrage du signal HR vrai comprend :
- définir un temps entre deux battements normal pendant une troisième période de temps;
- spécifier une fenêtre temporelle correspondant à la variation normale des temps entre deux battements pendant la troisième période de temps; et
- identifier tous les temps entre deux battements plus courts ou plus longs que la fenêtre temporelle comme battements cardiaques anormaux.

9. Produit de programme informatique selon la revendication 8, dans lequel la fenêtre temporelle est définie de manière dynamique sur la base des données HRV historiques du sujet.

10. Produit de programme informatique selon l'une des revendications 6 à 9, qui amène le système ou dispositif informatique également à effectuer les étapes consistant à :
obtenir (200) le signal ballistocardiographique du sujet en provenance d'un accéléromètre mesurant l'accélération dans la direction longitudinale du sujet.
